# EUROPEAN PATENT APPLICATION

(11) **EP 3 205 330 A1**
(43) Date of publication of application: **16.08.2017**
(21) Application number: 17155562.6
(22) Date of filing: 10.02.2017
(51) Int. Cl.: A61K 8/34, A61K 8/60, A61Q 9/04

(54) **EPILATION COMPOSITION AND ITS USE**

(30) Priority: 10.02.2016 FI 20165101
(71) Applicant: Suomen Hoitolatukku Tuonti Oy, 21310 Vahto (FI)
(72) Inventor: KUJALA, Harri, 21310 VAHTO (FI); MERIHARJU, Henna, 21310 VAHTO (FI)
(74) Representative: Berggren Oy, Turku

(57) **Abstract**

The invention relates to an epilation composition for removal of hair, comprising at least 75 weight-% of at least one sugar selected from sucrose, fructose and glucose, and ≤ 25 weight-% of xylitol.

## Description

The invention relates to an epilation composition and its use according to the preambles of the enclosed independent claims.

Naturally hair grows all over normal adult human body. In many cultures the hair growth at certain body areas is considered undesired or repulsive. The perception of socially acceptable hairiness may vary between males and females, and may change as time passes. Generally, female facial hair is often removed for social reasons. In western culture both female and male visible hair growth not only in armpits and legs but all over the body is generally considered unwanted. Also religion may impose regulations in regard of acceptable hair growth. For example, according to Islam all body hair should be completely removed regularly in shorter than 40 day intervals from all over the body excluding eye brows and men's beards. Furthermore, in some sports body hair is often removed from exposed parts of the body. For example, many professional cyclists remove their leg and derriere hair and swimmers remove hair from their legs, arms and torsos. Bodybuilders, bikini fitness and body fitness enthusiasts as well as models are also among people who remove their body hair periodically or on regular basis.

There exists a numerous different hair removing methods. In depilation, i.e. removal of hair, wool or bristles by chemical or mechanical methods, the hair above skin surface are removed, e.g. by shaving. In epilation the visible hair together with at least part of its root is removed. Epilation provides significantly longer hair-free period than depilation. By epilation the hair-free period may last up till several weeks.

Epilation may be performed by sugaring. In sugaring a sugar paste is applied onto the skin at room temperature or at slightly elevated temperature near the room temperature. The paste is applied onto the skin in opposite direction of the hair growth. The hairs are attached to the paste and then the paste is removed, with the hairs and their roots, in the direction of the hair growth. Epilation by sugaring provides many benefits in comparison by waxing. There is no need to warm the sugaring paste to high temperatures, so risk for burns during hair removal is avoided. Furthermore, sugaring causes less pain and is less irritating for the skin as the hair is removed in the direction to the growth, unlike waxing, where the hair is removed against the direction of the growth.

However, sometimes there is a possibility for irritation of the skin, even when the hair is removed by sugaring. It is possible that the hair follicles may become slightly irritated or inflamed, at least for very sensitive subjects. The skin irritation can be seen as small red dots or spots in the skin, which is at least a visual drawback. Consequently, there is a need for epilation composition which would reduce the risk for skin irritation or inflammation during and after epilation.

An object of this invention is to minimise or even eliminate the disadvantages existing in the prior art.

An object for the present invention is an epilation composition, which reduces the risk for skin irritation and/or follicle inflammation.

Another object of the present invention is an epilation composition which is safe, efficient and easy to use.

Yet another object of the present invention is an epilation composition which has an improved consistency, which attaches to the hair tightly.

These objects are attained with the invention having the characteristics presented below in the characterising parts of the independent claims. Some preferable embodiments are described in the dependent claims. All the described embodiments and advantages apply both for the epilation composition and the uses according to the present invention, when applicable, even if not always explicitly stated so.

A typical epilation composition according to the present invention for removal of hair, comprises
- at least 75 weight-% of at least one sugar selected from sucrose, fructose and glucose, and
- ≤ 25 weight-% of xylitol.

Typical use of composition according to the present invention is for removal of human body hair.

Typical use of xylitol according to the present invention is in an epilation composition for reducing skin irritation.

Now it has been surprisingly found that addition of xylitol to a sugar-based epilation composition reduces clearly the observed skin irritation after sugaring. The theoretical background of the invention is yet not fully understood, but without wishing to be bound by a theory it is assumed that xylitol has some kind of an anti-inflammatory effect and prevents the exposure of the follicles to bacteria. It seems that xylitol provides an antibacterial effect, which protects against and reduces possible bacterial or fungal infections in follicles. Furthermore, xylitol has a positive effect on consistency of the epilation composition, by making it more viscous. This means that the composition sticks to the hair tighter than conventional epilation compositions. Stronger attachment of the composition to the hair will make the epilation procedure more efficient and complete, as well as fast. It is assumed that the viscous epilation composition attaches predominantly onto hairs and dead skin cells on the skin surface, rather than living skin cells. This further strengthens the irritation decreasing effect obtainable by the epilation composition according to the present invention. Thus the epilation composition according to the present invention provides an unexpected synergetic effect of improved viscosity and anti-inflammatory effect that clearly reduce the risk of skin irritation or follicle inflammation.

In the present context xylitol denotes a sugar alcohol C₅H₁₂O₅, which is also known as (2R, 4S)-pentane-1,2,3,4,5-pentol. The melting temperature of the xylitol is in the range of 92 - 96 °C.

Xylitol can be added to the epilation composition during its manufacture by simple addition. Xylitol behaves similarly to the sugars, which are used in in the epilation composition, which makes it addition to the composition uncomplicated. No supplementary processing steps or devices are required.

According to one embodiment of the invention the composition comprises at least 1 weight-%, preferably at least 2 weight-%, more preferably at least 2.5 weight-%, even more preferably at least 5 weight-% of xylitol. The composition preferably comprises less than 25 weight-% of xylitol. If the amount of xylitol is more than 25 weigh-%, the viscosity of the epilation composition decreases in a manner that reduces it usability. The composition may comprise, for example, 1 - 25 weight-%, preferably 2 - 15 weight-%, more preferably 2.5 - 12.5 weight-%, even more preferably 5 - 10 weight-% of xylitol.
the composition comprises at least 75 weight-% of at least one sugar selected from sucrose, fructose and glucose. The composition may comprise 75 - 99 weight-%, preferably 85 - 98 weight-%, more preferably 87.5 - 97.5 weight-%, even more preferably 90 - 95 weight-% of at least one sugar selected from sucrose, fructose and glucose.

The ingredients of the epilation composition are of food grade purity, and the composition is packed in sterile manner. Thus the biological degradation of the composition during storage is avoided and the risk for inflammation and/or allergic reactions is minimized.

According to one preferable embodiment the epilation composition comprises both glucose and fructose, i.e. the sugar in the composition is selected from glucose and fructose. Glucose and fructose are monosaccharides, having melting temperature around 103 - 105 °C for fructose and around 146 °C for glucose. The epilation composition, where the sugar consists of glucose and fructose may need gentle warming to a temperature around 25 - 35 °C before it is applied on the skin. The composition may comprise 35 - 55 weight-%, preferably 40 - 50 weight-% of glucose, and 35 - 55 weight-%, preferably 40 - 50 weight-% of fructose. Careful determined amounts of glucose and fructose guarantee epilation composition which is of uniform quality and easy to apply. The amount of glucose and fructose guarantee the stability of the composition during storage, and reduce the risk of crystallization, gumming and other undesired phenomenon, which otherwise may destroy the composition and shorten significantly its usable life.

According to one embodiment of the invention the ratio of glucose and fructose in the epilation composition is from 40:60 to 60:40.

According to another embodiment the epilation composition may comprise sucrose, i.e. the sugar in the composition is sucrose. The composition may comprise 75 - 95 weight-%, preferably 80 - 95 weight-%, more preferably 85 - 95 weight-% of sucrose. Sucrose is a disaccharide with melting temperature around 160 °C. According to one preferable embodiment, in addition to sucrose the epilation composition comprises weak organic acid, preferably a tribasic acid, such as citric acid, or the like. Preferably the weak organic acid is citric acid. The weak organic acid improves the consistency, i.e. viscosity, of the epilation composition.

The epilation composition is in form of viscous amorphic paste. The composition may have a consistency of a thick honey. The viscosity of the epilation composition depends on the type and amount of used sugar, as well as optional ingredients in the epilation composition.

The composition comprises water in addition of sugar(s), xylitol and optional other ingredients. The used water is pure distilled water.

The optional other ingredients may be a thickening agent and/or ethereal oil.

According to one embodiment the epilation composition may comprise at the most 10 weight-% of a thickening agent. The amount of the thickening agent is typically in the range of 0.5 - 7.5 weight-%, preferably from 1 - 5 weight-%. The thickening agent is preferably a natural gum, such as acacia gum, guar gum, xanthan gum or locust bean gum, more preferably acacia gum or guar gum. According to one preferable embodiment thickening agent is used when the sugar in the epilation composition consists of sucrose. In general, use of thickening agent improves, typically increases, the viscosity of the epilation composition and its attachment to the hairs.

The epilation composition may alternatively or in addition comprise ethereal oil(s), i.e. essential oils. The amount of ethereal oil in the epilation composition may be in the range of 0.5 - 6 weight-%, preferably 1 - 5 weight-%. The used ethereal oil may be of natural or synthetic origin, as long as it is water soluble ethereal oil. Ethereal oils of natural origin are preferred. The ethereal oil may be selected from oils of rose, argan, lavender, eucalyptus, ylang ylang, vanilla, sandalwood, mandarin orange, orange, lemon, grapefruit, lime, peppermint, cedar wood, rosehip, lemongrass, cinnamon, jasmine, patchouli, pine, bergamot, cassia, marjoram, spearmint, tea tree, ginger, rosewood, camphor, frankincense and any of their mixtures. Used natural ethereal oil is preferably of organic origin. In this manner the risk for residual amounts of pesticides and/or biocides in the composition can be minimized or completely avoided. This is advantageous when the composition comes into contact with skin and follicles. According to one embodiment of the invention the epilation composition is free of pesticides and/or biocides.

The epilation composition is water-soluble, which makes it easy to clean off and/or remove if necessary. Water-soluble means in this context that the epilation composition is fully dissolved without any remaining solids visible by eyes when it comes into contact with excess water.

The composition according to the present invention is used for removal of human body hair by epilation. The composition is applied onto the skin of the subject at room temperature or at slightly elevated temperature near the room temperature, as defined above. The composition is applied onto the skin in opposite direction of the hair growth. The hairs are attached to the composition. Then the composition is removed, with the attached hairs and their roots, in the direction of the hair growth.

All weight percentages in this application are calculated from the weight of the final composition, if not otherwise stated.

### EXPERIMENTAL

An embodiment of the invention is explained more closely in the following non-limiting example.

### Example 1

The tested epilation composition according to the present invention comprised glucose, fructose and 10 weight-% of xylitol. The composition was ready-to-use at room temperature without any application of heat.

The reference epilation composition was a commercial sugaring composition without xylitol.

The inventive composition was applied onto the first leg of a female test subject and the hairs were removed. The reference composition and was applied onto the second leg of the same test subject and the hairs were removed. The total number of test subjects was approximately 80. The compositions were applied and the hair removed by a trained beauty therapist/qualified nurse.

The results were evaluated visually by naked eye and by using skin analysis devices with maximum of x600 magnification. It was observed that the number of red spots was significantly lower and smaller on size on the legs for which the inventive test composition was used. Furthermore, the test subjects considered the removal procedure with the inventive epilation composition faster and more efficient.

Even if the invention was described with reference to what at present seems to be the most practical and preferred embodiments, it is appreciated that the invention shall not be limited to the embodiments described above, but the invention is intended to cover also different modifications and equivalent technical solutions within the scope of the enclosed claims.

## Claims

1. Epilation composition for removal of hair, comprising
- at least 75 weight-% of at least one sugar selected from a group comprising sucrose, fructose and glucose, and
- ≤ 25 weight-% of xylitol.

2. Epilation composition according to claim 1, **characterised in that** the composition comprises at least 1 weight-%, preferably at least 2 weight-%, more preferably at least 2.5 weight-%, even more preferably at least 5 weight-% of xylitol.

3. Epilation composition according to claim 1, **characterised in that** the composition comprises 1 - 25 weight-%, preferably 2 - 15 weight-%, more preferably 2.5 - 12.5 weight-% of xylitol, even more preferably 5 - 10 weight-% of xylitol.

4. Epilation composition according to claim 1, 2 or 3, **characterised in that** the composition comprises both glucose and fructose.

5. Epilation composition according to claim 4, **characterised in that** the composition comprises 35 - 55 weight-%, preferably 40 - 50 weight-% of glucose, and 35 - 55 weight-%, preferably 40 - 50 weight-% of fructose.

6. Epilation composition according to claim 4, **characterised in** the ratio of glucose to fructose in the epilation composition is from 40:60 to 60:40.

7. Epilation composition according to claim 1, 2 or 3, **characterised in that** the composition comprises 75 - 95 weight-%, preferably 80 - 95 weight-%, more preferably 85 - 95 weight-% of sucrose.

8. Epilation composition according to any of preceding claims 1 - 7, **characterised in that** the epilation composition is in form of viscous paste.

9. Epilation composition according to any of preceding claims 1 - 8, **characterised in that** the composition comprises at the most 10 weight-% of a thickening agent, which is preferably a natural gum, such as acacia gum, guar gum or locust bean gum.

10. Epilation composition according to any of preceding claims 1 - 9, **characterised in that** the epilation composition comprises ethereal oil(s).

11. Epilation composition according to claim 10, **characterised in that** the amount of ethereal oil is in the range of 0.5 - 6 weight-%, preferably 1 - 5 weight-%.

12. Epilation composition according to any of preceding claims 1 - 11, **characterised in that** the epilation composition is water-soluble.

13. Use of composition according to any of claims 1 - 12 for removal of human body hair.

14. Use of xylitol in an epilation composition for reducing skin irritation.

15. Use according to claim 14, **characterised in that** the amount of xylitol in the epilation composition is at least 1 weight-%, preferably at least 2 weight-%, more preferably at least 2.5 weight-%, even more preferably at least 5 weight-%.
